# EUROPÄISCHE PATENTSCHRIFT

(11) **EP 2 400 944 B1**
(45) Veröffentlichungstag und Bekanntmachung des Hinweises auf die Patenterteilung: **28.12.2016**
(21) Anmeldenummer: 10704908.2
(22) Anmeldetag: 16.02.2010
(51) Int. Cl.: A61K 8/11, A61Q 19/10

(54) **STABILISIERTE PEELINGKAPSEL**
STABILIZED PEELING CAPSULE
CAPSULE DE GOMMAGE STABILISÉE

(30) Priorität: 24.02.2009 DE 102009010091
(43) Veröffentlichungstag der Anmeldung: 04.01.2012
(73) Patentinhaber: Beiersdorf Aktiengesellschaft, 20253 Hamburg (DE)
(72) Erfinder: THILKER, Janina, 22303 Hamburg (DE); HOFFMANN, Nils, 22303 Hamburg (DE)
(86) Internationale Anmeldenummer: PCT/EP2010/000950
(87) Internationale Veröffentlichungsnummer: WO 2010/097174

(56) Entgegenhaltungen:
- WO-A1-2005/020940
- WO-A1-2005/020949
- WO-A1-2008/034549
- WO-A1-2008/034565
- DE-A1-102007 019 373
- DE-U1-202007 002 529
- US-A1- 2006 127 427
- US-A1- 2009 017 110

## Beschreibung

Die Erfindung umfasst eine topisch applizierbare Kapsel umfassend ein Hüllmaterial und ein Füllmaterial enthaltend eine abrasiv wirkende Zubereitung, wobei die Hülle mittels Sprühtechnik oder Tauchbad mit einem Überzug versehen ist.

Die WO 2008034549 beschreibt topisch applizierbare und auf der Haut verreibbare Kapseln umfassend ein Hüllmaterial auf Emulsionsbasis und ein Füllmaterial enthaltend eine abrasiv wirkende Zubereitung umfassend ein oder mehrere abrasiv wirkende Peelingmittel.

Die kapselförmigen Zubereitungen sind als Dragees, Kapseln, Kugeln oder Hohlkugeln bei Lagerung und Entnahme formstabil.

Die WO 2008034565 beschreibt kosmetische und dermatologische Peelingzubereitungen in Form von Kapseln umfassend eine formstabile Kapselhülle und eine Füllung.

Ein zusätzlicher Überzug ist in beiden Dokumenten nicht offenbart

Die DE 102007019373 A1 offenbart sogenannte Mikrokapseln.

Die Mikrokapseln sind nach im Stand der Technik bekannten Verfahren zugänglich. Als Mikrokapseln lassen sich sämtliche auf dem Markt angebotenen tensidstabilen Mikrokapseln einsetzen, beispielsweise die Handelsprodukte (in Klammern angegeben ist jeweils das Hüllmaterial) Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose); Unicerin C30 (Lactose, mikrokristalline Cellulose, Hydroxypropylmethylcellulose), Kobo Glycospheres (modifizierte Stärke, Fettsäureester, Phospholipide), Softspheres (modifiziertes Agar Agar) und Kuhs Probiol Nanospheres (Phospholipide).

In DE 1027847, DE 2305280, DE 2406021 und DE 1924647 werden bekannte oral applizierbare Arzneimittelformen mit Lackbeschichtungen, wasserlösliche Hartschalenkapseln, Hartgelantinekapseln und magensaftresitente gelantinekapseln offenbart.

Keines der dargelegten Stand der Technik Dokumente konnte jedoch den Weg zur vorliegenden Erfindung weisen.

Zur Verbesserung der Lagerstabilität und Bruchfestigkeit von Peelingkapseln können Polymere auf die Hülle der Kapseln aufgetragen werden. Geeignete Polymere sind Celluloseether, Polyvinylpyrrolidon, Polyacrylate oder Polymethacrylate sowie Eudragit wie in US 2006127427 beschrieben.

US 2006127427 beschreibt topisch applizierbare und auf der Haut verreibbare Kapseln umfassend ein Hüllmaterial und ein Füllmaterial enthaltend eine abrasiv wirkende Zubereitung umfassend ein oder mehrere wasserlösliche Peelingpartikel wie Salz oder Zucker. Als Überzug der Kapsel werden polymerhaltige, zunächst wasserunlösliche, dann jedoch wasserlösliche Substanzen wie insbesondere Eudragit® (Acrylatcppolymer) beschrieben. Das Coatingmaterial Eudragit® löst sich pH-abhängig auf und gibt dann den Kern der Kapseln frei.

Die Kapseln können aus dem Stand der Technik bekannt mit Polier- oder Hartwachsen überzogen werden. Dazu zählen vor allem Schellack-, Carnauba- oder Bienenwachs sowie weitere Lackwachse.

Unter Dragieren versteht man das Umhüllen eines Kerns mit beispielsweise Zuckerschichten. Heute werden immer häufiger andere Überzugsmaterialien verwendet. Die aufgetragene Schicht besteht vornehmlich entweder aus Zucker (klassisches Zuckerdragee) oder aus einem anderen Filmbildner (Filmtabletten). Die Schicht ist meist gefärbt und kann gegebenenfalls noch andere Substanzen enthalten, um die Eigenschaften der fertigen Arzneiform in der gewünschten Weise zu verändern, wie beispielsweise den Geruch oder den Geschmack.

Zuckerdragierung ist das klassische Dragierverfahren, d.h. das Überziehen der Kerne mit Zuckerlösungen. Hohen Produktionskosten, die Schwierigkeiten den Prozess zu automatisieren und die lange Herstellungsdauer von bis zu einer Woche pro Charge lassen dieses Verfahren zugunsten der Filmtablette immer weiter zurücktreten.

Beim Kaltdragieren wird die Zuckerlösung bei normaler Zimmertemperatur aufgetragen, beim Warmdragieren (Heißdragieren) wird ein erwärmter Zuckersirup verwendet (ca. 50 - 60° C).

Der Vorgang findet in Dragierkesseln statt, in der die Kerne, die Kapseln durch Rotation der Trommel ins Rollen gebracht werden. Die Dragierflüssigkeit wird zugesetzt und überzieht nach und nach die Kerne. Gleichzeitig wird vorsichtig getrocknet, beispielsweise mit Warmluft oder UV-Strahler. Bei wärmeempfindlichen Substanzen kann auch über die Zufuhr von Kaltluft getrocknet werden.

Der Vorgang wird so lange wiederholt bis sich eine ausreichend dicke und stabile Schicht um den Kern gebildet hat. Dies kann bis zu 50 Dragiervorgänge erfordern. Die Kerne erfahren dadurch eine Größen- und Volumenzunahme.

Die Schnelldragierung entspricht in ihren wesentlichen Arbeitsgängen der oben beschriebenen Dragierung. Eine Zeitersparnis wird häufig dadurch realisiert, dass man sich mit einer um 70% - 90% geringeren Schichtdicke zufrieden gibt. Ferner werden zum Schnelldragieren Dragieremulsionen verwendet. In wenigen Stunden lassen sich so Dragees herstellen.

Nachteilig ist, dass die in Dragierkesseln überzogenen Kapseln für die Anwendung der Dragiertechnik eine ausreichende Eigenstabilität aufweisen und fest genug gegen äußere mechanische Schläge sein müssen. Anderenfalls kann es zu Verformungen und Verklumpungen der Kapseln während des Dragiervorganges im Kessel kommen.
Da es sich bei den aufgetragenen Zuckerlösungen um wässrige Lösungen handelt, die auf der Kapseloberfläche trocknen, darf die Kapselhülle nach der Dragierung kein Wasser abgeben bzw. muss am besten wasserfrei sein, da sich die Dragierung ansonsten leicht wieder ablöst und nicht fest um die Kapsel umschlossen ist. Die Kapseloberfläche würde dann feucht und vom aufgelösten Zucker klebrig werden.

Wünschenswert ist es neben wasserfreien Wachskapselhüllen auch wasserhaltige Emulsionshüllen zu dragieren bzw. durch einen Überzug zu stabilisieren.

Erfindungsgemäße Lösung ist eine topisch applizier- und verreibbare Kapsel umfassend ein wasserhaltiges Hüllmaterial und ein vom Hüllmaterial umschlossenes Füllmaterial, umfassend eine abrasiv wirkende Zubereitung, umfassend ein oder mehrere abrasiv wirkende Peelingmittel. Die erfindungsgemäße Kapsel ist gegenüber den kosmetischen Kapseln des Standes der Technik jedoch mit einem Überzug versehen, der mittels eines Tauchbades und/oder mittels Sprühtechnik auf die Kapsel aufgetragen ist.

Das Hüllmaterial ist bevorzugt aus einer Emulsion aufgebaut, umfassend ein oder mehrere Wachse, die oberhalb 25°C fest sind.

Die topisch applizierbare Kapsel ist bei Raumtemperatur eine feste, halbfeste bzw. formstabile Kapsel, die damit einzeln portionier- und handhabbar ist.

Viele Begriffe wie "Kugeln", "Kapseln", "kapselförmige Zubereitung" oder "Perlen" können grundsätzlich zur Beschreibung der erfindungsgemäßen Kapseln herangezogen werden, auch wenn diesen Begriffen unter Umständen verschiedene Bedeutungen zugeordnet werden. Insbesondere die Bedeutung des Begriffes "Kapsel" ist hierin nicht auf die genau definierten Formen, Herstellungsverfahren, Inhaltsstoffe und Anwendungsmöglichkeiten der ebenfalls "Kapseln" genannten pharmazeutischen Präparate beschränkt, schließt diese aber mit ein. Generell ist erfindungsgemäß eine Kapsel ein beispielsweise ungefähr runder oder ellipsoider, von seiner Umgebung klar unterscheidbarer Gegenstand, der bei leichtem Druck und beispielsweise durch Anfassen bei der Entnahme aus einer Verpackung, seine Form nur unwesentlich ändert.
Die kapselförmigen erfindungsgemäßen Zubereitungen können beliebige Formen haben, bevorzugt sind sie jedoch sphärisch mit einem Volumen von 0,1 bis 20 ml.
Die erfindungsgemäßen Kapseln weisen eine Größe, d.h. durchschnittlichen Durchmesser, von 3, bevorzugt 5, bis 40 mm auf. Damit sind die Kapseln einzeln handhabbar und anwendbar.
Die erfindungsgemäßen kapselförmigen Zubereitungen sind als Dragees, Kapseln, Kugeln, Perlen oder Hohlkugeln bei Lagerung und Entnahme formstabil.

Bevorzugt sind die in den WO 2008034565 und WO 2008034549 beschriebenen Kapseln als Ausgangszubereitungen zu verwenden. Der Offenbarungsgehalt der Druckschriften WO 2008034549 und WO 2008034565 ist vollumfänglich auch Offenbarungsgehalt der vorliegenden Erfindung.

Vorteilhaft besteht das Hüllmaterial der Kapsel nicht aus Gelatine oder reinem Wachs sondern ist emulsionsbasierend aufgebaut.

Da wasserhaltige Kapselhüllen nicht so stabil, wie beispielsweise eine reine Wachshülle, und dadurch anfälliger gegenüber mechanischen Einflüssen sind, werden die
erfindungsgemäßen Kapseln mit Hilfe einer Sprühtechnik oder mittels Tauchbad überzogen. Da diese Art des Überzuges nicht in einem Dragierkessel stattfindet und die Kapseln hierbei getrennt voneinander auf einer ebenen Unterfläche liegen bzw. durch ein ethanolisches Bad gezogen werden, kann die Verklumpung und das Zerbrechen der einzelnen Kapseln während des Dragiervorganges vermieden werden.

Gegenüber dem Stand der Technik sind die erfindungsgemäßen Peelingkapseln daher nicht nach bekannten Verfahren zu dragieren, wie in Dragierkesseln, sondern sie werden in Tauchbädern oder mittels Sprühtechnik mit einem Überzug versehen.

Bei der Auftragung des Überzugs mit Hilfe der Sprühtechnik werden die Kapseln nach der Herstellung auf einer ebenen Fläche, wie ein Förderband, gelegt und/oder transportiert und von oben mit einer ethanolischen Lösung besprüht. Die Feinversprühung kann mit Druckluft oder als Aerosol mit Treibgas, wie bei industriellen Sprüh-Coatings, die zur Beschichtung von Oberflächen angewendet werden, erfolgen. Durch beispielsweise einen Luftstrom wird die aufgetragene Lösung dann getrocknet. Dabei verdampft der Ethanol und zurück bleibt ein dünner stabilisierender Film auf der Kapsel. Durch mechanische Bewegung oder beispielsweise einen Luftstrom werden die Kapseln gedreht und erneut von den anderen Seiten besprüht und getrocknet.

Bei der Herstellung eines Überzuges durch ein Tauchbad durchlaufen die Kapseln nach der Herstellung ein Tauchbad aus beispielsweise ethanolischer Filmbildnerlösung oder einer ethanolischen Lacklösung. Nach dem Tauchbad wird der stabilisierende Überzug ebenfalls vorteilhaft durch einen leichten Luftstrom getrocknet.

Beim Antrocknen bzw. Verdampfen der Lösungsmittel ist es auch vorteilhaft, die Kapseln leicht zu drehen, damit die Überzugslösung auf allein Teilen der Kapsel gleichmäßig antrocknet.

Der erfindungsgemäße Überzug ist vorteilhaft eine ethanolische Filmbildnerlösung umfassend Octylacrylamide/Acrylates/Butylaminoethyl Methacrylate Copolymere oder Acrylates/Octylacrylamide Copolymer als Filmbildner.

Vorteilhaft sind auch andere Filmbildner-Polymere erfindungsgemäß einsetzbar wie Schellack (Shellac Cera), Carnauba- oder Bienenwachs

Erfindungsgemäß wird auf den Einsatz von Zucker in der Hülle als auch dem Überzug verzichtet.

Der Überzug ist vorteilhaft wasserunlöslich, was gegenüber den zahlreichen Dokumenten des Standes der Technik ein mitentscheidenden Unterschied darstellt.

Vorteilhaft und zur verbesserten Haftfähigkeit des aufgetragenen Überzugs auf die Emulsionshülle der erfindungsgemäßen Peelingkapsel wird diese vor der Sprüh- bzw. Tauchbehandlung zunächst angeraut. Dazu wird erfindungsgemäß eine dünne pulverförmige Schicht auf die Hülle aufgetragen. Die pulverförmige Schicht kann aus pulverförmigen Wachsen, Disacchariden, Polysacchariden, Cellulose oder Gummi arabicum und/oder Perlglanzpigmenten bestehen.

Die Pulverauftragung erfolgt nach der Erstellung der Kapseln und vor dem zuvor beschriebenen Auftrag des Überzugs.
Zum Pulverauftrag werden die Kapseln beispielsweise auf einer ebenen Unterfläche oder dem Förderband liegend von oben mit Pulver bestäubt oder berieselt. Dieser Berieselungsvorgang kann vergleichbar wie das Verfahren, bei dem Pralinen mit Schokolade überzogen werden, erfolgen. Dabei befinden sich die Pralinen, hier Peelingkapseln, auf einem Laufband und an einer Stelle wird von oben Schokolade auf die Pralinen gegossen, hier Pulver auf die Peelingkapsel. Auf dem Transportband/-bahn (auch im Kühltunnel) trocknet der Überzug. Dabei sollten die Kapseln bevorzugt gleichzeitig für eine gleichmäßige Auftragung des Pulvers, beispielsweise durch einen Luftstrom von unten, gedreht bzw. bewegt werden.
Die Pulverauftragung oder -beaufschlagung kann auch in einer Art kleinem Dragierkessel erfolgen, in dem jedoch aufgrund der leichten Verformbarkeit der Kapseln nur wenige oder nur eine Kapseln gleichzeitig angeraut werden sollte. Hierbei werden die Kapseln gedreht und gleichzeitig in Pulver gewälzt.
Für ein optisch ansprechenderes Aussehen kann der pulverförmigen Schicht Perlglanz, Farbpigmente oder andere bunte Pulverpartikel zugefügt werden, die die Oberfläche der Kapseln einfärben. Die Pulverschicht wird anschließend durch den beschriebenen Überzug fixiert.

Die aufgetragene Pulverdicke ist vorteilhaft höchstens 0,5 mm dick. Die Pulverbeschichtung dient in erster Linie zum leichten Aufrauen bzw. Einfärben der Oberfläche der erfindungsgemäßen Kapsel.

Als Pulverschicht werden vorteilhafterweise Perlglanzpigmente, wie beispielsweise Silica + Mica + CI 77891 verwendet

Gegenüber den bekannten Überzügen hat der erfindungsgemäße Überzug den Vorteil, dass dieser Überzug auch bei wasserhaltigen Kapseln auf Emulsionsbasis anwendbar ist. Da hierbei kein Dragierkessel verwendet wird, ist das Verfahren auch für weiche Kapseln anwendbar.
Außerdem ist es mit Hilfe dieses Verfahrens des Pulverauftrags möglich einen optisch ansprechenden Perlglanz-/Schimmer-Effekt auf der Kapsel zu erzielen.

Durch die Behandlung mit einer Polymerlösung oder einem Wachs ist die Kapsel formstabiler und durch den getrockneten Überzug kann ein zusätzlicher Peelingeffekt der Kapsel erzielt werden.

Bei den Kapseln des Standes der Technik, wie beispielweise in US 2006127427 beschrieben, werden wasserlösliche Peelingpartikel wie Salz oder Zucker mit einem Coating ummantelt, das nicht wasserlöslich ist und zum Zerbrechen erst aktiviert werden muss (durch Änderung des pH-Wertes oder durch Aktivierung mit Wasser).
Das Coating stellt somit gleichzeitig auch die eigentliche Hülle dar und schützt die Peelingpartikel.
Zum Unterschied wird erfindungsgemäß eine wasserhaltige Hülle, vornehmlich emulsionsbasierend, mit einem zusätzlichen Filmcoating mittels Tauchbad oder Sprühtechnik geschützt.
Der Schutz der Hülle zur Stabilisierung der Peelingperlen bzw. der Schutz der Hülle und den sich daran befindlichen Perlglanzpigmenten steht erfindungsgemäß im Vordergrund.

Erfindungsgemäß wird eine portionierbare Darreichung eines Gesichtspeelings aufgrund der Kapselform angeboten. Die pflegenden und reinigenden Produkteigenschaften werden kombiniert in einem Produkt zur Verfügung gestellt.

Die erfindungsgemäße Kapsel ist damit zum gleichzeitigen Hautpeeling und zur Hautpflege zu verwenden.

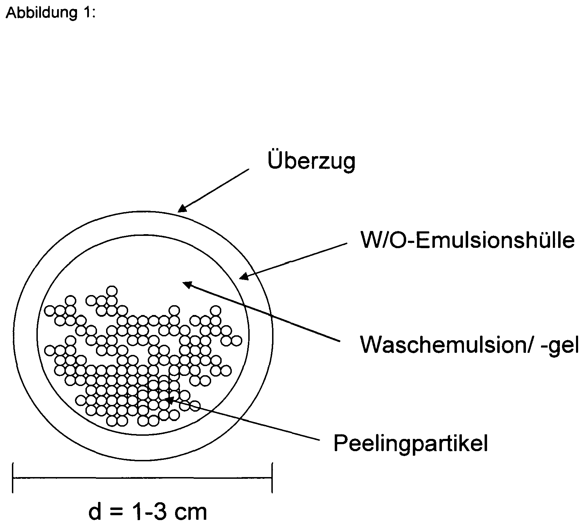

In Abbildung 1 ist ein Beispiel der erfindungsgemäßen Kapsel dargestellt.
Bevorzugt setzt sich die Kapsel aus einer
- W/O-Emulsionshülle und einer
- Waschemulsions-Füllung mit abrasiven Teilchen
zusammen.

Der Anteil der Hülle liegt vorteilhaft im Bereich von 35 Gew.% bis 65 Gew.%, bevorzugt im Bereich von 55 Gew.%, bezogen auf die Gesamtmasse der Kapsel.
Der Anteil der Füllung liegt vorteilhaft im Bereich von 35 Gew.% bis 65 Gew.%, insbesondere im Bereich von 45 Gew.%, bezogen auf die Gesamtmasse der Kapsel.

Vorteilhaft ist die Hülle aus einer W/O Emulsion aufgebaut.

Die Peelingmittel werden vorteilhaft gewählt aus der Gruppe Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von Walnusschalen, Aprikosen-, Pfirsich- und/oder Mandelkernen.

### Beispiele

Die Zahlenangaben beziehen sich auf Gewichtsprozent jeweils bezogen auf die Gesamtmasse der Hülle bzw. der Füllung.

### Zusammensetzung Hülle:

| **1** | **m [%]** |
|---|---|
| Cetyl Palmitate | 4,0000 |
| Glycerin | 5,0000 |
| Hexamidine Diisethionate | 0,0800 |
| Isopropyl Palmitate | 25,3200 |
| Octyldodecanol | 29,0000 |
| PEG-45/Dodecyl Glycol Copolymer | 1,3000 |
| Polyethylene | 13,0000 |
| Polyglyceryl-3 Diisostearate | 1,3000 |
| Tocopheryl Acetate | 1,0000 |
| Water | 20,0000 |

| | **m [%]** |
|---|---|
| Beeswax | 4,0000 |
| C20-40 Alkyl Stearate | 7,5000 |
| Caprylic/Capric Triglyceride | 12,0000 |
| Cetyl Palmitate | 12,0000 |
| Helianthus Annuus (Sunflower) Seed Wax | 4,0000 |
| Hydrogenated Coco-Glycerides | 45,0000 |
| Isopropyl Stearate | 12,0000 |
| Lanolin Alcohol (Eucerit®) | 0,5000 |
| Silica Dimethyl Silylate | 3,0000 |
| Water | ad |

### Zusammensetzung Füllung:

| 2. | **m [%]** |
|---|---|
| Acrylates/C10-30 Alkyl Acrylate Crosspolymer | 0,6000 |
| BHT | 0,0500 |
| Fragrance | 1,0000 |
| Glycine Soja (Soybean) Oil + Ricinus Communis (Castor) Seed Oil + Propyl Gallate | 9,0500 |
| Hydrolyzed Silk + Phenoxyethanol + Methylparaben + Ethylparaben + Butylparaben + Isobutylparaben + Propylparaben | 0,1000 |
| Lactose + Cellulose + Ultramarines + Hydroxypropyl Methylcellulose + Tocopheryl Acetate | 0,3000 |
| Lauryl Glucoside | 4,4700 |
| Methylparaben | 0,3500 |
| Mineral Oil | 35,0000 |
| Oenothera Biennis (Evening Primrose) Oil | 0,1000 |
| Phenoxyethanol | 0,8000 |
| Polyethylene | 4,3000 |
| Propylparaben | 0,3500 |
| Sodium Myreth Sulfate | 7,3200 |
| Water | 36,2100 |

### 3. Waschemulsion (mit Peelingpartikeln):

| | **m [%]** |
|---|---|
| Bis-Diglyceryl Polyacyladipate-2 | 1,5000 |
| Carbomer | 0,5000 |
| Ceteareth-20 | 2,0000 |
| Cetyl Alcohol | 2,2000 |
| Cetyl Palmitate | 1,0000 |
| Decyl Glucoside | 0,2500 |
| Decyl Oleate | 1,0000 |
| Fragrance | 0,3000 |
| Glycerin | 4,3500 |
| Glyceryl Stearate | 1,5000 |
| Glycine Soja (Soybean) Oil + Calendula Officinalis Flower Extract | 0,2000 |
| Isopropyl Palmitate | 7,5000 |
| Lactose + Cellulose + Ultramarines + Hydroxypropyl Methylcellulose + Tocopheryl Acetate | 0,2500 |
| Methylparaben | 0,2000 |
| Methylpropanediol | 2,0000 |
| Mineral Oil | 4,5000 |
| PEG-20 Glyceryl Stearate | 2,5000 |
| Phenoxyethanol | 0,6000 |
| Polyethylene | 4,3000 |
| Prunus Amygdalus Dulcis (Sweet Almond) Oil | 0,2000 |
| Water | 63,1500 |

### 4. Waschgel (mit Peelingpartikeln):

| | **m [%]** |
|---|---|
| Acrylates/C10-30 Alkyl Acrylate Crosspolymer | 0,6000 |
| BHT | 0,0500 |
| Fragrance | 1,0000 |
| Glycine Soja (Soybean) Oil + Ricinus Communis (Castor) Seed Oil + Propyl Gallate | 9,0500 |
| Hydrolyzed Silk + Phenoxyethanol + Methylparaben + Ethylparaben + Butylparaben + Isobutylparaben + Propylparaben | 0,1000 |
| Lauryl Glucoside | 4,4700 |
| Methylparaben | 0,3500 |
| Mineral Oil | 35,0000 |
| Oenothera Biennis (Evening Primrose) Oil | 0,1000 |
| Phenoxyethanol | 0,8000 |
| Polyethylene | 4,0000 |
| Polyethylene | 0,3000 |
| Propylparaben | 0,3500 |
| Sodium Myreth Sulfate | 7,3200 |
| Water | 36,5100 |

### 5. Zusammensetzung Überzug:

| **Gehalt** | |
|---|---|
| 96% | Alcohol Denat. |
| 3% | Octylacrylamide/Acrylates/Butylaminoethyl Methacrylate Copolymer |
| 1 % | Aminomethyl Propanol |

Die beispielhaften Füllungen und Hüllen bilden die Kapsel, die anschließend erfindungsgemäß mit dem Überzug mittels Sprühtechnik oder Tauchbad überzogen werden. Vorteilhaft wird vor dem Aufbringen des Überzugs eine Pulverschicht aufgetragen, um die Rauhigkeit und ggf. optische Akzeptanz zu verbessern.

## Patentansprüche

1. Topisch applizier- und verreibbare Kapsel umfassend
a. ein von einem Überzug umschlossenes wasserhaltiges Hüllmaterial und
b. ein vom Hüllmaterial umschlossenes Füllmaterial, umfassend
c. eine abrasiv wirkende Zubereitung, umfassend
d. ein oder mehrere abrasiv wirkende Peelingmittel,
**dadurch gekennzeichnet, dass**
der Überzug mittels Sprühtechnik oder Tauchbad auf die Hülle aufgebracht wird und die Hülle und/oder der Überzug keinen Zucker umfasst

2. Kapsel nach Anspruch 1 dadurch gekennzeichnte, dass das Hüllmaterial aus einer Emulsion aufgebaut ist, umfassend ein oder mehrere Wachse, die oberhalb 25°C fest sind.

3. Kapsel nach Anspruch 1 oder 2 **dadurch gekennzeichnet, dass** die Peelingmittel gewählt werden aus der Gruppe Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von Walnusschalen, Aprikosen-, Pfirsich- und/oder Mandelkernen.

4. Kapsel nach einem der vorstehenden Ansprüche **dadurch gekennzeichnet, dass** der Überzug wasserunlöslich ist.

5. Kapsel nach einem der vorstehenden Ansprüchen **dadurch gekennzeichnet, dass** auf der Hülle eine pulverförmige Schicht aufgetragen ist, wobei der Pulverauftrag vor dem Auftrag des Überzugs erfolgt.

6. Kapsel nach Anspruch 5 **dadurch gekennzeichnet, dass** die Pulverschicht maximal 0,5 mm dick ist.

7. Kapsel nach einem der Ansprüchen 5 oder 6 **dadurch gekennzeichnet, dass** die Pulverschicht Wachse, Disaccharide, Polysaccharide Cellulose, Gummi arabicum und/oder Perlglanzpigmente umfasst.

## Claims

1. Topically applicable and grindable capsule comprising
a) a water-containing coating material surrounded by a film and
b) a filling material surrounded by the coating material, comprising
c) an abrasive preparation, comprising
d) one or more abrasive peeling agents,
**characterized in that**
the film is applied to the coating by means of a spraying technique or dipping bath and the coating and/or the film does not comprise any sugar.

2. Capsule according to Claim 1, **characterized in that** the coating material is composed of an emulsion comprising one or more waxes which are solid above 25°C.

3. Capsule according to Claim 1 or 2, **characterized in that** the peeling agents are selected from the group of polyethylene, table salt, sea salt, sodium carbonate, sodium hydrogen carbonate, magnesium sulphate, magnesium chloride, sugar, clay earth, sand, plastic particles, pounded or ground cores of walnut shells, apricot, peach and/or almond kernels.

4. Capsule according to any of the preceding claims, **characterized in that** the film is insoluble in water.

5. Capsule according to any of the preceding claims, **characterized in that** a powdery layer is applied to the coating, wherein the powder application takes place before the application of the film.

6. Capsule according to Claim 5, **characterized in that** the powder layer is a maximum of 0.5 mm thick.

7. Capsule according to Claims 5 or 6, **characterized in that** the powder layer comprises waxes, disaccharides, polysaccharides, cellulose, gum arabic and/or pearlescent pigments.

## Revendications

1. Capsule pouvant être appliquée et étalée par voie topique, comprenant
a. une matière d'enveloppe contenant de l'eau, entourée par un revêtement et
b. une matière de remplissage, entourée par la matière d'enveloppe, comprenant
c. une préparation à action abrasive, comprenant
d. un ou plusieurs exfoliants à action abrasive,
**caractérisée en ce que** le revêtement est appliqué au moyen d'une technique de pulvérisation ou d'un bain d'immersion sur l'enveloppe et l'enveloppe et/ou le revêtement ne contien(nen)t pas de sucre.

2. Capsule selon la revendication 1, **caractérisée en ce que** la matière d'enveloppe est formée à partir d'une émulsion, comprenant une ou plusieurs cires qui sont solides au-dessus de 25°C.

3. Capsule selon la revendication 1 ou 2, **caractérisée en ce que** les exfoliants sont choisis dans le groupe constitué par le polyéthylène, le chlorure de sodium, le sel de mer, le carbonate de sodium, l'hydrogénocarbonate de sodium, le sulfate de magnésium, le chlorure de magnésium, le sucre, l'alumine, le sable, des particules de matière plastique, des grains concassés ou broyés de coquilles de noix, de noyaux d'abricot, de pêche et/ou de coques d'amandes.

4. Capsule selon l'une quelconque des revendications précédentes, **caractérisée en ce que** le revêtement est insoluble dans l'eau.

5. Capsule selon l'une quelconque des revendications précédentes, **caractérisée en ce qu'**une couche sous forme de poudre est appliquée sur l'enveloppe, l'application de la poudre ayant lieu avant l'application du revêtement.

6. Capsule selon la revendication 5, **caractérisée en ce que** la couche de poudre présente une épaisseur maximale de 0,5 mm.

7. Capsule selon l'une quelconque des revendications 5 ou 6, **caractérisée en ce que** la couche de poudre comprend des cires, des disaccharides, des polysaccharides, de la cellulose, de la gomme arabique et/ou des pigments nacrés.
